# EUROPÄISCHE PATENTSCHRIFT

(11) **EP 0 796 251 B1**
(45) Veröffentlichungstag und Bekanntmachung des Hinweises auf die Patenterteilung: **17.10.2001**
(21) Anmeldenummer: 95941683.5
(22) Anmeldetag: 07.12.1995
(51) Int. Cl.: C07D 239/54, C07D 239/60, C07D 285/16, A61K 31/505

(54) **URACILDERIVATE**
URACIL DERIVATIVES
DERIVES D'URACILE

(30) Priorität: 07.12.1994 CH 370994
(43) Veröffentlichungstag der Anmeldung: 24.09.1997
(73) Patentinhaber: Byk Gulden Lomberg Chemische Fabrik GmbH, 78467 Konstanz (DE)
(72) Erfinder: BOER, Rainer, D-78464 Konstanz (DE); BOSS, Hildegard, D-78464 Konstanz (DE); ELTZE, Manfrid, D-78462 Konstanz (DE); BELLER, Klaus-Dieter, D-78465 Konstanz (DE); SANDERS, Karl, D-78464 Konstanz (DE); BRAND, Ursula, D-78462 Konstanz (DE); PRÜSSE, Wolfgang, D-78476 Allensbach (DE); ULRICH, Wolf-Rüdiger, D-78464 Konstanz (DE)
(86) Internationale Anmeldenummer: EP9504802
(87) Internationale Veröffentlichungsnummer: WO9617831

(56) Entgegenhaltungen:
- DE-A- 3 326 118
- US-A- 3 957 786

## Beschreibung

Die Erfindung betrifft Verbindungen, die in der pharmazeutischen Industrie als Wirkstoffe für die Herstellung von Arzneimitteln verwendet werden sollen.

### Bekannter technischer Hintergrund

Im US-Patent 3,957,786 werden substituierte Uracilderivate beschrieben, die sich insbesondere durch eine ausgeprägte blutdrucksenkende Wirkung auszeichnen.

### Beschreibung der Erfindung

Überraschenderweise wurde nun gefunden, daß die nachfolgend näher beschriebenen Verbindungen ausgeprägte Affinität für 5-HT_{1A}-Rezeptoren und eine differenzierte Affinität für Subtypen der α₁-Adrenozeptoren besitzen.

Gegenstand der Erfindung sind Verbindungen der Formel I (siehe beiliegendes Formelblatt), worin
- R1: 1-4C-Alkyl bedeutet,
- R2: 1-4C-Alkyl bedeutet,
- R3: Wasserstoff (H), 1-7C-Alkyl, 3-7C-Alkenyl, 1-4C-Alkoxy, Halogen, Halo-1-4C-alkyl, Cyan-1-4C-alkyl, 1-4C-Alkoxycarbonyl, Nitro, Hydroxyiminomethyl, Methoxyiminomethyl oder eine Gruppe -CH₂-RI bedeutet, worin RI den in der Formel I an den Substituenten R3 gebundenen Rest bedeutet,
- A: einen geradkettigen oder verzweigten 1-5C-Alkylenrest bedeutet,
- Ar: einen durch R4, R5 und R6 substituierten Phenylrest bedeutet, worin
R4 Wasserstoff, Halogen, Nitro, Trifluormethyl, Hydroxy, 1-4C-Alkoxy, ganz oder teilweise durch Fluor substituiertes 1-4C-Alkoxy, Amino oder 1-4C-Alkylcarbonylamino bedeutet,
R5 Wasserstoff, Halogen oder 1-4C-Alkoxy bedeutet und
R6 Wasserstoff oder 1-4C-Alkoxy bedeutet, oder worin
R4 und R5 zueinander orthoständig sind und gemeinsam einen 1-Hydroxymethyl-ethylendioxyrest [-O-CH(CH₂OH)-CH₂-O-] bedeuten und
R6 Wasserstoff bedeutet.
- X: die Gruppierung NH oder CO-NH bedeutet,
- Y: Sauerstoff (O) oder Schwefel (S) bedeutet und
- Z: CO oder SO₂ bedeutet,
und ihre Salze,
wobei
- R3: nicht Wasserstoff (H), 1-7C-Alkyl, Halogen oder Nitro bedeutet, wenn
- R4: Wasserstoff, Halogen, 1-4C-Alkoxy oder Trifluormethyl,
- R5: Wasserstoff, Halogen oder 1-4C-Alkoxy,
- R6: Wasserstoff oder 1-4C-Alkoxy,
- X: die Gruppierung NH,
- Y: Sauerstoff (O) und
- Z: CO bedeutet.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

1-7C-Alkyl steht für geradkettige oder verzweigte Alkylreste mit 1 bis 7 Kohlenstoffatomen. Beispielsweise seien genannt der Heptyl-, Isoheptyl-(2-Methylhexyl-), Hexyl-, Isohexyl- (2-Methylpentyl-), Neohexyl- (2,2-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl-, Ethyl- und der Methylrest.

3-7C-Alkenyl steht für einen geradkettigen oder verzweigten Alkenylrest mit 3 bis 7 Kohlenstoffatomen. Als bevorzugte 3-7C-Alkenylreste seien der 2-Butenyl-, der 3-Butenyl-, der 1-Propenyl- und der 2-Propenylrest (Allylrest) genannt.

1-4C-Alkoxy steht für einen Rest, der neben dem Sauerstoffatom einen der vorstehend genannten 1-4C-Alkylreste enthält. Beispielsweise seien der Methoxy- und der Ethoxyrest genannt.

Halogen im Sinne der vorliegenden Erfindung ist Brom, Chlor und Fluor.

Halo-1-4C-alkyl steht für einen der vorstehend genannten 1-4C-Alkylreste, der durch eines der vorstehend genannten Halogenatome substituiert ist. Beispielsweise sei der 3-Chlorpropylrest genannt.

Cyan-1-4C-alkyl steht für einen der vorstehend genannten 1-4C-Alkylreste, der durch eine Cyangruppe substituiert ist. Beispielsweise sei der 2-Cyanethylrest genannt.

1-4C-Alkoxycarbonyl steht für einen Rest, der neben der Carbonylgruppe einen der vorstehend genannten 1-4C-Alkoxyreste enthält. Beispielsweise seien der Methoxycarbonyl- und der Ethoxycarbonylrest genannt.

Hydroxyiminomethyl steht für den Rest -CH=N-OH, Methoxyiminomethyl für den Rest -CH=N-OCH₃.

Wenn R3 die Bedeutung -CH₂-RI hat, so liegt eine Verbindung der Formel RI-CH₂-RI vor, worin RI den in der Formel I an den Substituenten R3 gebundenen Rest bedeutet.

Geradkettiges oder verzweigtes 1-5C-Alkylen ist beispielsweise Methylen (-CH₂), Ethylen (-CH₂-CH₂-), Trimethylen (-CH₂-CH₂-CH₂-), Tetramethylen (CH₂-CH₂-CH₂-CH₂-), 1,2-Dimethylethylen [-CH(CH₃)-CH(CH₃)-], 1,1-Dimethylethylen [-C(CH₃)₂-CH₂-], 1,1-Dimethylpropylen [-C(CH₃)₂-CH₂-CH₂-], 2,2-Dimethylethylen [-CH₂-C(CH₃)₂-], Isopropyliden [-C(CH₃)₂-] und 1-Methylethylen [-CH(CH₃)-CH₂-]. Bevorzugt ist die Gruppe Trimethylen (Propylen).

Als ganz oder teilweise durch Fluor substituiertes 1-4C-Alkoxy seien beispielsweise der 1,2,2-Trifluorethoxy, der 2,2,3,3,3-Pentafluorpropoxy-, der Perfluorethoxy- und insbesondere der 1,1,2,2-Tetrafluorethoxy-, der Trifluormethoxy-, der 2,2,2-Trifluorethoxy- und der Difluormethoxyrest genannt.

1-4C-Alkylcarbonyl steht für einen Rest, der neben der Carbonylgruppe einen der vorstehend genannten 1-4C-Alkylreste enthält. Beispielsweise sei der Acetylrest genannt.

1-4C-Alkylcarbonylamino steht für eine Aminogruppe, die durch einen der vorstehend genannten 1-4C-Alkylcarbonylreste substituiert ist. Beispielsweise sei der Acetamidorest genannt.

Wenn R4 und R5 gemeinsam einen 1-Hydroxymethyl-ethylen-dioxyrest bedeuten, so liegt - gemeinsam mit dem Phenylrest, an den R4 und R5 gebunden sind - ein 2,3-Dihydro-2-hydroxymethyl-1,4-benzodioxanylrest vor, wobei sich die Substituenten R4 und R5 bevorzugt in 2- und 3-Position zur Phenylbindungsstelle befinden, so daß bevorzugt ein 2,3-Dihydro-2-hydroxymethyl-1,4-benzodioxan-5-ylrest vorliegt. Bei diesem Rest handelt es sich um einen chiralen Rest. Die Erfindung umfaßt sowohl die Enantiomeren als auch Mischungen der Enantiomeren in jedem beliebigen Mischungsverhältnis, einschließlich der Racemate. Bevorzugt ist der 2,3-Dihydro-2-hydroxymethyl-1,4-benzodioxan-5-ylrest, bei dem im chiralen Kohlenstoffatom die Substituenten (gemäß Cahn, Ingold und Prelog) in S-Konfiguration angeordnet sind.

Als Salze kommen für Verbindungen der Formel I vor allem alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Besonders erwähnenswert sind solche Verbindungen der Formel I, worin R1 1-4C-Alkyl bedeutet,
- R2: 1-4C-Alkyl bedeutet,
- R3: Wasserstoff (H), 1-4C-Alkyl, 3-4C-Alkenyl, 1-4C-Alkoxy, Cyan-1-4C-alkyl, 1-4C-Alkoxycarbonyl, Hydroxyiminomethyl, Methoxyiminomethyl oder eine Gruppe -CH₂-RI bedeutet, worin RI den in der Formel I an den Substituenten R3 gebundenen Rest bedeutet,
- A: einen geradkettiqen 2-4C-Alkylenrest bedeutet.
- Ar: einen durch R4, R5 und R6 substituierten Phenylrest bedeutet, worin
R4 Wasserstoff, Halogen, Nitro, Hydroxy, 1-4C-Alkoxy, ganz oder teilweise durch Fluor substituiertes 1-4C-Alkoxy, Amino oder 1-4C-Alkylcarbonylamino bedeutet,
R5 Wasserstoff, Halogen oder 1-4C-Alkoxy bedeutet und
R6 Wasserstoff oder 1-4C-Alkoxy bedeutet, oder worin
R4 und R5 zueinander orthoständig sind und gemeinsam einen 1-Hydroxy-methyl-ethylendioxyrest [-O-CH(CH₂OH)-CH₂-O-] hedeuten und
R6 methyl-ethylendioxyrest [-O-CH(CH₂OH)-CH₂-O-] bedeuten und Wasserstoff bedeutet,
- X: die Gruppierung NH oder CO-NH bedeutet,
- Y: Sauerstoff (O) oder Schwefel (S) bedeutet und
- Z: CO oder SO₂ bedeutet,
und ihre Salze,
wobei
- R3: nicht Wasserstoff (H) oder 1-4C-Alkyl bedeutet, wenn
- R4: Wasserstoff, Halogen oder 1-4C-Alkoxy,
- R5: Wasserstoff, Halogen oder 1-4C-Alkoxy,
- R6: Wasserstoff oder 1-4C-Alkoxy,
- X: die Gruppierung NH,
- Y: Sauerstoff (O) und
- Z: CO bedeutet.

Weiterhin sind solche Verbindungen der Formel I besonders erwähnenswert, worin
- R1: 1-4C-Alkyl bedeutet,
- R2: 1-4C-Alkyl bedeutet,
- R3: Wasserstoff (H), 1-4C-Alkyl, 3-4C-Alkenyl, 1-4C-Alkoxy, Cyan-1-4C-alkyl, 1-4C-Alkoxycarbonyl, Hydroxyiminomethyl, Methoxyiminomethyl oder eine Gruppe -CH₂-RI bedeutet, worin RI den in der Formel I an den Substituenten R3 gebundenen Rest bedeutet,
- A: einen Trimethylenrest bedeutet,
- Ar: einen durch R4, R5 und R6 substituierten Phenylrest bedeutet, worin
R4 Wasserstoff, Halogen, Nitro, Hydroxy, 1-4C-Alkoxy oder ganz oder teilweise durch Fluor substituiertes 1-4C-Alkoxy bedeutet,
R5 Wasserstoff, Halogen oder 1-4C-Alkoxy bedeutet und
R6 Wasserstoff oder 1-4C-Alkoxy bedeutet, oder worin
R4 und R5 zueinander orthoständig sind und gemeinsam einen 1-Hydroxymethyl-ethylendioxyrest [-0-CH(CH₂OH)-CH₂-O-] bedeuten und
R6 Wasserstoff bedeutet,
- X: die Gruppierung NH oder CO-NH bedeutet,
- Y: Sauerstoff (O) bedeutet und
- Z: CO bedeutet,
und ihre Salze,
wobei
- R3: nicht Wasserstoff (H) oder 1-4C-Alkyl bedeutet, wenn
- R4: Wasserstoff, Halogen oder 1-4C-Alkoxy,
- R5: Wasserstoff, Halogen oder 1-4C-Alkoxy,
- R6: Wasserstoff oder 1-4C-Alkoxy und
- X: die Gruppierung NH bedeutet.

Weiterer Gegenstand der Erfindung ist ein Verfahren zur Herstellung der Verbindungen der Formel I, das dadurch gekennzeichnet ist, daß man
a) Verbindungen der Formel II (siehe beiliegendes Formelblatt), worin R1, R2, R3, Y und Z die oben angegebenen Bedeutungen haben und M eine Abgangsgruppe (z.B. -Br oder -Cl) oder eine an eine Carbonylgruppe gebundene Abgangsgruppe (z.B. -CO-Br oder -CO-Cl) bedeutet, mit Verbindungen der Formel III (siehe beiliegendes Formelblatt), worin A und Ar die oben angegebenen Bedeutungen haben umsetzt, oder daß man
b) Verbindungen der Formel IV (siehe beiliegendes Formelblatt), worin R1, R2, R3, A, X, Y und Z die oben angegebenen Bedeutungen haben und L eine Abgangsgruppe (z.B. ein Halogenatom) bedeutet, mit Verbindungen der Formel V (siehe beiliegendes Formelblatt), worin Ar die oben angegebene Bedeutung hat, umsetzt, oder daß man
c) Verbindungen der Formel I, in denen R3 Wasserstoff bedeutet, mit solchen reaktiven Verbindungen umsetzt, die - unter Abspaltung dieses Wasserstoffatoms in einer Kondensationsreaktion, oder unter Addition dieses Wasserstoffatoms an eine Doppelbindung - den gewünschten, von Wasserstoff verschiedenen Substituenten R3 ergeben, oder daß man
d) zur Herstellung der Verbindungen I, in denen R3 die Gruppe -CH₂-RI bedeutet, Verbindungen der Formel I, in denen R3 Wasserstoff bedeutet, mit Formaldehyd umsetzt, oder daß man
e) zur Herstellung der Verbindungen I, in denen R3 Hydroxyiminomethyl bedeutet, Verbindungen der Formel I, in denen R3 -CH=0 bedeutet, mit Hydroxylamin umsetzt, oder daß man
f) zur Herstellung der Verbindungen I, in denen R3 Methoxyiminomethyl bedeutet, Verbindungen der Formel I, in denen R3 Hydroxyiminomethyl bedeutet, methyliert, oder daß man
g) zur Herstellung der Verbindungen I, in denen Y Schwefel (S) bedeutet, Verbindungen der Formel I, in denen Y Sauerstoff (0) bedeutet, sulfidiert, oder daß man
h) zur Herstellung der Verbindungen I, in denen R4 Amino bedeutet, Verbindungen der Formel I, in denen R4 Nitro bedeutet, reduziert, oder daß man
i) zur Herstellung der Verbindungen I, in denen R4 1-4C-Alkylcarbonylamino bedeutet, Verbindungen der Formel I, in denen R4 Amino bedeutet, acyliert,
und daß man gewünschtenfalls anschließend erhaltene Verbindungen der Formel I in ihre Salze überführt, oder daß man erhaltene Salze in die freien Verbindungen überführt.

Die Durchführung des Verfahrens entsprechend den Varianten a) bis i) erfolgt als Analogieverfahren auf eine dem Fachmann an sich bekannte Weise.

Die Umsetzung erfolgt jeweils in geeigneten, inerten Lösungsmitteln, in Gegenwart eventuell erforderlicher Hilfsreagenzien (wie beispielsweise Hilfsbasen, etwa bei den Varianten a, b oder i) und bei der für die jeweilige Reaktion zweckmäßigen Temperatur.

Welche Lösungsmittel und Hilfsreagenzien im einzelnen infrage kommen, bei welchen Temperaturen zu arbeiten ist und welche Reaktionszeiten eingehalten werden müssen, ist dem Fachmann aufgrund seines Fachwissens geläufig. Die nachfolgenden Beispiele dienen hierzu als exemplarische Erläuterung.

### Beispiele

### 1. 1,3,5-Trimethyl-6-[[3-[4-(3,4-dibrom-6-methoxyphenyl)-1-piperazinyl]propyl]amino]-2,4(1H,3H)-pyrimidindion

3,8 g (20 mmol) 1,3,5-Trimethyl-6-chlor-2,4(1H,3H)-pyrimidindion werden mit 9,6 g (20 mmol) 1-(3-Aminopropyl)-4-(3,4-dibrom-6-methoxyphenyl)-piperazin und 15,2 ml (80 mmol, 11,5 ml) Tripropylamin in 40 ml Dimethylsulfoxid 5 h auf 140°C erwärmt.

Nach Aufnehmen mit 600 ml Essigester, waschen mit 200 ml 1 N NaOH, zweimal mit 400 ml Wasser und 200 ml NaCl-gesättigtem Wasser wird die organische Phase mit MgSO₄ getrocknet und eingedampft. Der Rückstand wird säulenchromatographisch über 400 g Kieselgel (Fließmittel erst Essigester, später Essigester/Methanol = 10:1) gereinigt. Man erhält auf diese Weise 3,0 g (27 %) der Titelverbindung vom Schmp. 153-155°C.

Das Vorprodukt 1-(3-Aminopropyl)-4-(3,4-dibrom-6-methoxyphenyl)-piperazin ist erhältlich durch Bromierung von 1-(2-Methoxyphenyl)-piperazin mit Brom in a) konz. H₂SO₄ und anschließend b) in Eisessig, Umsetzung mit N-(3-Brompropyl)phthalimid zu 1-[3-(N-Phthalimidopropyl)]-4-(3,4-dibrom-6-methoxyphenyl)piperazin (Schmp. 158-161°C) und Spaltung mit Hydrazinhydrat (Zersetzungstemperatur des Dioxalats 204°C).

### 2. Bis[1,3-dimethyl-6-[[3-[4-(2-methoxyphenyl)-1-piperazinyl]propyl]amino]-2,4(1H,3H)-pyrimidindion-5,5']methan

7,75 g (20 mmol) 1,3-Dimethyl-6-[[3-[4-(2-methoxyphenyl)-1-piperazinyl]propyl]amino]-2,4(1H,3H)-pyrimidindion werden mit 50 ml eines Ethanol/Wasser-Gemisches (80:20) und 6,4 ml (80 mmol) einer 36,3 %igen wäßrigen Formaldehyd-Lösung versetzt und 5 h bei 50°C gerührt. Nach Zugabe von 0,4 g KOH wird weitere 2 h bei 50°C gerührt, die Lösung im Vakuum bis zum zäh-öligen Rückstand eingeengt, mit 50 ml Wasser und 100 ml Dichlormethan extrahiert, die organische Phase mit NaCl-Lösung gewaschen, über Na₂SO₄ getrocknet, eingeengt und der Rückstand aus Essigester umkristallisiert. Man erhält auf diese Weise 6,6 g (83 %) der Titelverbindung vom Schmp. 133-136°C.

Die Herstellung des als Ausgangsprodukt dienenden 1,3-Dimethyl-6-[[3-[4-(2-methoxyphenyl)-1-piperazinyl]propyl]amino]-2,4(1H,3H)-pyrimidindions ist in der DE-PS 1 942 405 beschrieben.

### 3. 5-Cyanethyl-1,3-Dimethyl-6-[[3-[4-(2-ethoxyphenyl)-1-piperazinyl]propyl]amino]-2,4(1H,3H)-pyrimidindion

4,3 g (15 mmol) 6-(3-Chlorpropylamino)-5-cyanethy-1,3-dimethyl-2,4(1H,3H)-pyrimidindion und 4,2 g (15 mmol) 1-(2-Ethoxyphenyl)piperazin werden in 50 ml Xylol mit 11,4 ml (60 mmol) Tripropylamin 14 h auf 140°C erhitzt. Das Reaktionsgemisch wird im Vakuum eingeengt, der Rückstand mit 50 ml Wasser und 200 ml Essigester aufgenommen, mit 300 ml 1 N NaOH versetzt, extrahiert und getrennt. Der organische Extrakt wird mit gesättigter NaCl-Lösung gewaschen, getrocknet und das nach Entfernen des Lösungsmittels erhaltene Öl säulenchromatographisch gereinigt (Kieselgel neutral, Fließmittel anfangs Essigester/Petrolether 1:1, später nur Petrolether). Aus den Produktfraktionen erhält man in einer Ausbeute von 57 % die Titelverbindung vom Schmp. 133-136°C (aus Ether).

Das Vorprodukt 6-(3-Chloropropylamino)-5-cyanethyl-1,3-dimethyl-2,4(1H,3H)-pyrimidindion wird durch Reaktion von 6-(3-Chloropropylamino)-1,3-dimethyl-2,4(1H,3H)-pyrimidindion mit Acrylnitril erhalten (Aceton/K₂CO₃, 18 h 60°C, Ausbeute 50 %, Schmp. 122-124°C).

### 4. Bis[1,3-dimethyl-6-[[3-[4-(2-ethoxy-4-fluorphenyl)-1-piperazinyl]propyl]amino]-2,4(1H,3H)-pyrimidindion-5,5']methan

Analog zu Beispiel 2 erhält man aus 1,3-Dimethyl-6-[[3-[4-(2-ethoxy-4-fluorphenyl)-1-piperazinyl]propyl]amino]-2,4(1H,3H)-pyrimidindion mit Formaldehyd und KOH (Molverhältnis 1:5:1) nach 20 h Rühren bei RT die Titelverbindung in einer Ausbeute von 76 % vom Schmp. 141-143°C (aus Essigester).

Das als Ausgangsprodukt eingesetzte 1,3-Dimethyl-6-[3-[4-(2-ethoxy-4-fluorphenyl)1-piperazinyl]propyl]amino]-2,4(1H,3H)-pyrimidindion wird analog dem im Beispiel 2 genannten Ausgangsprodukt hergestellt (Ausbeute 56 %, Schmp. 148-150°C).

### 5. 1,3-Dimethyl-2,4(1H,3H)-pyrimidindion-6-carbonsäure-[[3-[4-(2-methoxyphenyl)-1-piperazinyl]propyl]amid]

3,5 g (20 mmol) 1,3-Dimethyl-2,4(1H,3H)-pyrimidindion-6-carbonsäure werden zur Herstellung des Säurechlorids mit 20 ml Thionylchlorid 1 h am Rückfluß erhitzt. Das überschüssige Thionylchlorid wird im Vakuum abdestilliert. Der Rückstand wird in 10 ml Dichlormethan gelöst und unter Eiskühlung werden 5 g (20 mmol) 1-(2-Methoxyphenyl)-4-(3-aminopropyl)piperazin und 3 g (30 mmol) Triethylamin in 20 ml Dichlormethan zugetropft. Nach 18 h bei RT werden 150 ml Wasser, 20 ml 2 N NaOH und 50 ml Dichlormethan zugegeben, die organische Phase wird abgetrennt, mit Wasser gewaschen, getrocknet und im Vakuum das Lösungsmittel entfernt. Aus dem Rückstand erhält man 3,6 g (43 %) der Titelverbindung vom Schmp. von 96-99°C (nach Umfällen aus Wasser/Ethanol = 4:1 und Umkristallisieren aus Toluol/Ether; die Substanz enthält 0,5 Mol Wasser).

Die Herstellung der 1,3-Dimethyl-2,4(1H,3H)-pyrimidindion-6-carbonsäure ist beschrieben von M. P. Groziak im J. Am. Chem. Soc. 104, 6434 (1982).

### 6. 1,3,5-Trimethyl-6-[[3-[4-(2-tetrafluorethoxyphenyl)-1-piperazinyl]propyl]aminol-2,4(1H,3H)-pyrimidindion

Analog zum Beispiel 1 wird durch Einsatz von 6-Chlor-1,3,5-trimethyl-2,4-(1H,3H)-pyrimidindion und 1-(3-Aminopropyl)-4-(2-tetrafluorethoxyphenyl)-piperazin mit Triethylamin in 20 ml Ethylenglykolmonomethylether (16 h Rückfluß) die Titelverbindung in einer Ausbeute von 15 % vom Schmp. 120-124°C erhalten.

Das Vorprodukt 1-(3-Aminopropyl)-4-(2-tetrafluorethoxyphenyl)piperazin erhält man analog zu dem im Beispiel 1 beschriebenen Vorprodukt durch Spaltung des 1-(N-Phthalimidopropyl)-4-(2-tetrafluoroethoxyphenyl)piperazins nach bekannter Methode (Ausbeute 35 %, Zersetzungstemperatur des Dihydrochlorids, das ein Mol Wasser enthält, 153°C).

### 7. 5-Allyl-1,3-dimethyl-6-[[3-[4-(2-ethoxy-4-fluorphenyl)-1-piperazinyl]-propyl]amino]-2,4(1H,3H)-pyrimidindion

Analog zu Beispiel 3 wird durch Reaktion von 5-Allyl-1,3-dimethyl-6-(3-chlorpropylamino)-2,4(1H,3H)-pyrimidindion mit 1-(2-Ethoxy-4-fluorphenyl)-piperazin in Xylol und Tripropylamin (14 h Rückfluß) nach säulenchromatographischer Reinigung die Titelverbindung vom Schmp. 103-105°C in einer Ausbeute von 55 % erhalten.

Das Ausgangsprodukt 5-Allyl-1,3-dimethyl-6-(3-chlorpropylamino)-2,4(1H,3H)-pyrimidindion erhält man durch Reaktion von 1,3-Dimethyl-6-(3-chloropropylamino)-2,4(1H,3H)-pyrimidindion mit Allylbromid (Aceton/K₂CO₃, 70 h 60°C), Ausbeute 80%, Schmp. 74-76°C.

Das Hydrochlorid des 1-(2-Ethoxy-4-fluorphenyl)piperazins wird aus 2-Ethoxy-4-fluoranilin und Bis(2-chlorethyl)amin erhalten; Ausbeute 56 %, Schmp. 199-200°C.

### 8. 5-Cyanethyl-1,3-dimethyl-6-[[3-[4-(2-tetrafluorethoxyphenyl)-1-piperazinyl]propyl]amino]-2,4(1H,3H)-pyrimidindion

Analog zum Beispiel 3 erhält man die Titelverbindung aus 5-Cyanethyl-6-(3-chlorpropylamino)-1,3-dimethyl-2,4(1H,3H)-pyrimidindion mit 1-(2-Tetrafluorethoxyphenyl)piperazin. Ausbeute 41 %, Schmp. 130-132°C.

Das als Ausgangsprodukt dienende 1-(2-Tetrafluorethoxyphenyl)-piperazin wird aus 2-Tetrafluorethoxyanilin und Bis(2-chlorethyl)amin erhalten (Ausbeute an öligem Hydrochlorid 44 %).

### 9. 1,3,5-Trimethyl-6-[[3-r4-((2,3-dihydro-2-hydroxymethyl)-1,4-benzodioxin-5-yl)-1-piperazinyl]propyl]amino]-2,4(1H,3H)-pyrimidindion

Analog zum Beispiel 1 wird unter Einsatz von 1-(3-Aminopropyl)-4-((2,3-dihydro-2-hydroxymethyl)-1,4-benzodioxin-5-yl)piperazin, das durch Spaltung der entsprechenden Phthalimidoverbindung erhalten wird (Ausbeute 73 %, Öl), die Titelverbindung hergestellt (Ausbeute 61 %, Schmp. 119-122°C).

1-(3-Aminopropyl)-4-((2,3-dihydro-2-hydroxymethyl)-1,4-benzodioxin-5-yl)piperazin ist erhältlich aus 1-((2,3-Dihydro-2-hydroxymethyl)-1,4-benzodioxin-5-yl)piperazin (dessen Benzoesäureester in EP 138280 beschrieben ist) analog zur Herstellung des Vorproduktes des Beispiels 1 mit N-(3-Brompropyl)phthalimid und Spaltung mit Hydrazinhydrat (Ausbeute 73 %).

### 10. Bis[1,3-dimethyl-6-[[3-[4-((2,3-dihydro-2-hydroxymethyl)-1,4-benzodioxin-5-yl)-1-piperazinyl]propyl]amino]-2,4(1H,3H)pyrimidindion-5,5']methan

Analog zu Beispiel 2 oder 4 erhält man durch Einsatz von 1,3-Dimethyl-6-[[3-[4-((2,3-dihydro-2-hydroxymethyl)-1,4-benzodioxin-5-yl)-1-piperazi-nyl]propyl]amino]-2,4(1H,3H)-pyrimidindion die Titelverbindung in einer Ausbeute von 83 % mit einem Schmp. von 134-137°C.

Die Ausgangsverbindung ist durch Reaktion von 6-(3-Chlorpropylamino)-1,3-dimethyl-2,4(1H,3H)-pyrimidindion mit 1-((2,3-Dihydro-2-hydroxymethyl)-1,4-benzodioxin-5-yl)piperazin zu erhalten (Ausbeute 55 %, Zersetzungstemperatur des Trihydrochlorids 208°C).

### 11. 5-Allyl-1,3-dimethyl-6-[[3-[4-(2-tetrafluorethoxyphenyl)-1-piperazinyl]propyl]amino]-2,4(1H,3H)-pyrimidindion

Nach der Methode des Beispiels 3 werden das Pyrimidindion des Beispiels 7 und das Piperazin des Beispiels 8 umgesetzt. Die Titelverbindung wird in einer Ausbeute von 25 % mit einer Zersetzungstemperatur des Fumarats von 145°C erhalten.

### 12. 1,3-Dimethyl-5-ethoxycarbonyl-6-[[3-[4-(2-methoxyphenyl)-1-piperazinyl]propyl]amino]-2,4(1H,3H)-pyrimidindion

Die Reaktion der Ausgangsverbindung des Beispiels 2 mit Chloressigsäureethylester (Suspension in Pyridin, erst 0°C, dann 1 h bei 22°C, dann 0,5 h Rückfluß, Reinigung säulenchromatographisch) ergibt die Titelverbindung in 39 %iger Ausbeute vom Schmp. 104-106°C.

### 13. 1,3,5-Trimethyl-6-[[3-[4-(2-hydroxyphenyl)-1-piperazinyl]propyl]amino]-2,4(1H,3H)-pyrimidindion

Das aus 6,8 g (30 mmol) 1,3,5-Trimethyl-6-(3-hydroxypropylamino)2,4(1H,3H)-pyrimidindion mit Thionylchlorid hergestellte 1,3,5-Trimethyl-6-(3-chlorpropylamino)-2,4(1H,3H)-pyrimidindion wird mit 4,5 g (25 mmol) 1-(2-Hydroxyphenyl)piperazin in 140 ml Xylol und 20 ml Tripropylamin 15 h am Rückfluß gekocht. Das nach üblicher Aufarbeitung erhaltene dunkle Öl wird säulenchromatographisch über Kieselgel mit Dichlormethan/Methanol (1-10 %) gereinigt. Aus den Produktfraktionen wird aus Essigester/Ethanol mit Fumarsäure das Fumarat gefällt, Ausbeute 5,9 g (47 %), Schmp. 203-205°C.

Die Ausgangsverbindung 1-(2-Hydroxyphenyl)piperazin wird durch Spaltung von 1-(2-Methoxyphenyl)piperazin mit Pyridin-Hydrochlorid nach bekanntem Verfahren (Molverhältnis 1:10, 4 h 180°C) in einer Ausbeute von 92 % vom Schmp. 125-128°C erhalten.

### 14. 1,3,5-Trimethyl-6-[[3-[4-(2-methoxy-4-nitrophenyl)-1-piperazinyl]propyl]amino]-2,4(1H,3H)-pyrimidindion

Analog zum Beispiel 13 wird 1,3,5-Trimethyl-6-(3-chlorpropylamino)-2,4(1H,3H)-pyrimidindion (130 mmol) mit 1-(2-Methoxy-4-nitrophenyl)piperazin (100 mmol) und Tripropylamin (75 ml) in 300 ml Diglyme 14 h am Rückfluß gekocht. Nach Entfernen des Lösungsmittels im Vakuum wird wie üblich aufgearbeitet und nach säulenchromatographischer Reinigung erhält man die Titelverbindung in 79 %iger Ausbeute (Schmp. 131-133°C).

1-(2-Methoxy-4-nitrophenyl)piperazin wird erhalten nach Nitrierung von 1-Acetyl-4-(2-methoxyphenyl)piperazin mit 33 %iger Salpetersäure in Eisessig und anschließender Hydrolyse durch Kochen mit konz. Salzsäure, Ausbeute 58 %, Schmp. 89-91°C.

### 15. 1,3-Dimethyl-5-methoxyiminomethyl-6-[[3-[4-(2-methoxyphenyl)-1-piperazinyl]propyl]amino]-2,4(1H,3H)-pyrimidindion

20,1 g (43,5 mmol) 1,3-Dimethyl-5-hydroxyiminomethyl-6-[[3-[4-(2-methoxyphenyl)-1-piperazinyl]propyl]amino]-2,4(1H,3H)-pyrimidindion werden in 100 ml Ethanol gelöst, 11,6 ml (61 mmol) 30 %ige Natriummethylat-Lösung und 5,8 ml (7,7 g, 61 mmol) Dimethylsulfat zugeben und es wird 2 h am Rückfluß erhitzt. Nach Abdestillieren des Ethanols im Vakuum wird mit 100 ml Wasser und 50 ml 1 N NaOH versetzt und dreimal mit 75 ml Dichlormethan extrahiert; die vereinigten Extrakte werden über Magnesiumsulfat getrocknet und der Eindampfrückstand wird aus 50 ml Ethanol kristallisiert. Auf diese Weise werden 14,6 g (76 %) der Titelverbindung mit einem Schmp. 107-109°C erhalten.

### 16. 1,3-Dimethyl-5-hydroxyiminomethy]-6-[[3-[4-(2-methoxyphenyl)-1-piperazinyl]propyl]amino]-2,4(1H,3H)-pyrimidindion

Aus 1,3-Dimethyl-5-formyl-6-[[3-[4-(2-methoxyphenyl)-1-piperazinyl]propyl]-amino]-2,4(1H,3H)-pyrimidindion, das aus 1,3-Dimethyl-6-[[3-[4-(2-methoxyphenyl)1-piperazinyl]propyl]amino]-2,4(1H,3H)-pyrimidindion durch Reaktion mit POCl₃/DMF nach Vilsmeier-Haak erhalten wird (Ausbeute 78 %, Schmp. 121-123°C), wird die Titelverbindung mit Hydroxylamin-Hydrochlorid/Natriumacetat in Ethanol (2 h Rückfluß) erhalten. Ausbeute nach Umkristallisation aus Methanol 48 % des Methanol-Adduktes, Zersetzungstemperatur ab 96°C.

### 17. 1,3-Dimethyl-5-methoxy-6-[3-(4-[2-methoxyphenyl]-1-piperazinyl)-propyl]amino-2,4-(1H,3H)-pyrimidindion

Auf analoge Weise wie in Beispiel 1 beschrieben, erhält man aus 1,3-Dimethyl-5-methoxy-6-chlor-2,4(1H,3H)-pyrimidindion und 1-(3-Aminopropyl)-4-(2-methoxyphenyl)-piperazin die Titelverbindung in 79 %iger Ausbeute vom Schmp. 112-113°C.

### 18. 5-{3-[4-(2-Methoxy-phenyl)-piperazin-1-yl]-propylamino}-2,6-dimethyl-1,1-dioxo-6H-[1,2,6]thiadiazin-3-on

Analog zum Beispiel 1 wird unter Verwendung von 120 mmol 6H-2,3-Dihydro-2,6-dimethyl-3-oxo-5-chlor-1,2,6-thiadiazine-1,1-dioxide und 240 mmol 1-(2-Methoxyphenyl)-4-(3-aminopropyl)piperazin durch 1,5 stündiges Schmelzen bei 125°C und nach Reinigung mittels Säulenchromatographie über Kieselgel und Umkristallisieren aus Essigester die Titelverbindung vom Schmp. 137-139°C erhalten (Ausbeute 36 %).

Die Ausgangsverbindung 6H-2,3-Dihydro-2,6-dimethyl-3-oxo-5-chlor-1,2,6-thiadiazin-1,1-dioxid ist erhältlich durch Chlorierung der 5-Hydroxyverbindung mit POCl₃/H₃PO₄ (2 h Rückfluß), Ausbeute 40 %, Schmp. 51-2°C. Die Herstellung der Hydroxyverbindung ist bekannt und erfolgt durch Umsatz von Dimethylsulfamid und Malonsäuredichlorid [P. Goya et al., Can. J. Chem. 65, 298 (1987)], Ausbeute 89 %, Schmp. 92-93°C.

### 19. 6-{3-[4-(2-Methoxy-phenyl)-piperazin-1-yl]-propylamino)-1,3-dimethyl-4-thioxo-3,4-dihydro-1H-pyrimidin-2-on

Nach der Methode von A. R. Lapucha, Synthesis 1987, 256 werden aus je 10 mmol 1,3-Dimethyl-6-[(3-[4-(2-methoxyphenyl)-1-piperazinyl]propyl)amino]-2,4(1H,3H)-pyrimidindion und Tetraphosphordecasulfid P₄S₁₀ in 15 ml Diglyme und 40 mmol NaHCO₃ (4 h erhitzen auf 140°C) 35 % der Titelverbindung vom Schmp. 171-174°C erhalten.

### 20. 1,3,5-Trimethyl-6-[[3-[4-(4-amino-2-methoxyphenyl)-1-piperazinyl]propyl]amino]-2,4(1H,3H)-pyrimidindion

50 mmol 1,3,5-Trimethyl-6-[(3-[4-(2-methoxy-4-nitrophenyl)-1-piperazinyl]-propyl)amino]-2,4(1H,3H)-pyrimidindion werden nach bekannter Methode in Ethanol/Wasser mit Hydrazinhydrat/Raney-Nickel reduziert. Man erhält nach säulenchromatographischer Reinigung und Umkristallisation aus Ethylacetat 73 % der Titelverbindung vom Schmp. 146-148°C. Die Ausgangssubstanz ist das Produkt des Beispiels 14.

### 21. 1,3,5-Trimethyl-6-[[3-[4-(4-acetylamino-2-methoxyphenyl)-1-piperazinyl]propyl]amino]-2,4(1H,3H)-pyrimidindion

Die Substanz des Beispiels 20 wird in bekannter Weise mit Essigsäureanhydrid acetyliert (1 h 110°C). Man erhält in einer Ausbeute von 28 % die Titelverbindung vom Schmp. 178-180°C.

### Gewerbliche Anwendbarkeit

Die Verbindungen der Formel I und ihre Salze besitzen wertvolle pharmakologische Eigenschaften, die sie gewerblich verwertbar machen. Sie weisen insbesondere eine hohe Affinität zu 5-HT_{1A}-Rezeptorbindungsstellen auf. Darüberhinaus zeichnen sie sich durch eine differenzierte α₁-Adrenozeptor-Subtyp-antagonistische Wirksamkeit aus. Trotz ihrer Affinität zur 5-HT_{1A}-Bindungsstelle und zu α₁-Rezeptor-Subtypen weisen sie aber nur eine vergleichsweise weniger stark ausgeprägte blutdrucksenkende Wirkung auf.

In ihren ausgezeichneten Eigenschaften, die verbunden sind mit einer großen therapeutischen Breite und dem Fehlen wesentlicher Nebenwirkungen, erweisen sich die erfindungsgemäßen Verbindungen und ihre Salze als besonders geeignet für den Einsatz in der Human- und Veterinärmedizin, wobei sie insbesondere zur Behandlung solcher Krankheiten eingesetzt werden können, die durch 5-HT_{1A}-Agonisten bzw. α₁-Adrenorezeptorenblocker therapierbar sind. In diesem Zusammenhang sind vor allem Krankheiten zu nennen, die auf zentralnervösen Störungen beruhen (z.B. akute und chronische Angstzustände, Depressionen, Anorexie, Sexualstörungen, Psychosen), Schlafstörungen, senile Störungen der mentalen Funktion (z.B. senile Demenz), cerebrale Ischämien, apoplexia cerebri, aber auch Krankheiten, die mit einer übersteigerten Vasokonstriktion (z.B. arterieller Bluthochdruck) oder einer erhöhten Muskelkontraktion in bestimmten Bereichen (z.B. neurogene Blasenstörungen, Prostatahypertrophie) einhergehen.

Ein weiterer Gegenstand der Erfindung sind daher die erfindungsgemäßen Verbindungen zur Anwendung bei der Behandlung und/oder Prophylaxe der vorstehend genannten Krankheiten.

Ebenso umfaßt die Erfindung die Verwendung der erfindungsgemäßen Verbindungen zur Herstellung von Arzneimitteln, die zur Behandlung und/oder Prophylaxe der vorstehend genannten Krankheiten eingesetzt werden.

Weiterhin umfaßt die Erfindung die Verwendung der erfindungsgemäßen Verbindungen zur Behandlung und/oder Prophylaxe der vorstehend genannten Krankheiten.

Ein weiterer Gegenstand der Erfindung sind Arzneimittel, die ein oder mehrere Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfs- oder Trägerstoffen in Form von Tabletten, Dragees, Kapseln, Suppositorien, Pflastern (z.B. als TTS), Emulsionen, Suspensionen oder Lösungen eingesetzt, wobei der Wirkstoffgehalt vorteilhafterweise zwischen 0,1 und 95 % beträgt und wobei durch die entsprechende Wahl der Hilfs- und Trägerstoffe eine auf den Wirkstoff und/oder auf den gewünschten Wirkungseintritt genau angepaßte galenische Darreichungsform (z.B. eine Retardform oder eine magensaftresistente Form) erzielt werden kann.

Welche Hilfs- bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,05 bis etwa 30, vorzugsweise 0,1 bis 10, insbesondere 0,2 bis 6 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxyd, Magnesiumaluminat; Tranquilizer, wie Benzodiazepine, beispielsweise Diazepam; Spasmolytika, wie z.B. Bietamiverin, Camylofin, Anticholinergica, wie z.B. Oxyphencyclimin, Phencarbamid; Lokalanaesthetika, wie z.B. Tetracain, Procain; gegebenenfalls auch Fermente, Vitamine oder Aminosäuren enthalten.

### Biologische Untersuchungen

### Bestimmung der Affinitäten von Substanzen zu α₁-Rezeptor-Subtypen und 5-HT_{1A}-Rezeptoren

Die Affinität von Substanzen zu α₁-Rezeptor-Subtypen wurde in Radioliganden Bindungsstudien mit [³H]Prazosin als Radioligand bestimmt. 50-200 *µ*g Membranen aus Rattenhirn-Kortex wurden in einem Gesamtvolumen von 0,5-1 ml mit [³H]Prazosin (0,2 bis 0,5 nmol/l) in Gegenwart steigender Konzentrationen an Substanzen inkubiert. Als Inkubationspuffer wurde 50 mmol/l Tris/HCl pH 7,5, 1 mmol/l EDTA, 0,1 mmol/l PMSF verwendet. Die unspezifische Bindung wurde in separaten Ansätzen in Gegenwart von 10 *µ*mol/l Phentolamin oder 1 *µ*mol/l Prazosin bestimmt. Nach 1 h bei 37°C wurde die gebundene Radioaktivität durch rasche Filtration des Inkubationsansatzes bei 0°C über Glasfaserfilter von der freien Radioaktivität getrennt. Die Filter wurden zweimal mit je 3,5 ml kaltem Filtrationspuffer (50 mmol/l Tris/HCl pH 7,4, 10 mmol/l MgCl₂, 10 % Polyethylenglykol 6000) gewaschen. Die filtergebundene Radioaktivität wurde durch Flüssigkeits-Szintillationszählung bestimmt. Substanzen mit Subtypselektivität für die α₁-Rezeptorsubtypen wurden erneut in Gegenwart von 3 nmol/l WB 4101 getestet. WB 4101 blockiert die Bindung von [³H]Prazosin zum α_{1A}-Rezeptorsubtyp und die verbleibende Bindung entspricht derjenigen am α_{1B}-Rezeptor.

Die Affinität von Substanzen zum 5-HT_{1A} -Rezeptor wurde in Radioliganden Bindungsstudien mit [³H]8-OH-DPAT als Radioligand bestimmt. 50-150 *µ*g Membranen aus Schweinehirn-Kortex wurden in einem Gesamtvolumen von 0,25-0,5 ml mit [³H]8-OH-DPAT (0,2 bis 0,4 nmol/l) in Gegenwart steigender Konzentrationen an Substanzen inkubiert. Als Inkubationspuffer wurde 50 mmol/l Tris/HCl pH 8,2, 1 mmol/l 1 MnCl₂ verwendet. Die unspezifische Bindung wurde in separaten Ansätzen in Gegenwart von 10 *µ*mol/l 5-HT bestimmt. Nach 30 min bei 23°C wurde die gebundene Radioaktivität durch rasche Filtration des Inkubationsansatzes bei 0°C über Glasfaserfilter von der freien Radioaktivität getrennt. Die Filter wurden zweimal mit je 3,5 ml kaltem Filtrationspuffer (50 mmol/l Tris/HCl pH 7,4, 10 mmol/l MgCl₂, 10 % Polyethylenglykol 6000) gewaschen. Die filtergebundene Radioaktivität wurde durch Flüssigkeits-Szintillationszählung bestimmt.

Für jede Substanz wurden Dosis-Antwortkurven mittels nichtlinearer Regression mit dem Inplot-Programm (GraphPad, Sorento, CAL, USA) erstellt. IC₅₀-Werte wurden um die eingesetzte Konzentration des Radioliganden nach der Formel von Cheng und Prusoff korrigiert. Die resultierenden K_{I}-Werte (siehe nachfolgende Tabelle) geben die Affinität der jeweiligen Substanz zum getesteten Rezeptor wieder. Die gemäß dem vorstehend näher erläuterten Modell untersuchten erfindungsgemäßen Verbindungen sind in der nachfolgenden Tabelle mit Nummern versehen worden, die den Nummern dieser Verbindungen in den Beispielen entsprechen.

**Tabelle**

| Affinität der erfindungsgemäßen Verbindungen zu α₁-Rezeptor-Subtypen und 5-HT_{1A}-Rezeptoren, ausgedrückt in pK_{I}-Werten | | | |
|---|---|---|---|
| Verbindung | Rezeptoraffinität | | |
| Nr. | α_{1A} | α_{1B} | 5-HT_{1A} |
| 1 | 8,3 | 8,3 | 8,9 |
| 2 | 9,4 | 9,4 | 7,9 |
| 3 | 9,3 | 7,2 | 9,4 |
| 4 | 9,0 | 9,0 | 7,4 |
| 5 | 8,3 | 6,6 | 8,6 |
| 6 | 9,4 | 7,5 | 9,7 |
| 7 | 9,2 | 7,4 | 9,1 |
| 8 | 9,2 | 7,5 | 10,0 |
| 9 | 8,7 | 5,8 | 9,7 |
| 10 | 8,1 | 8,1 | 9,3 |
| 11 | 9,6 | 7,9 | 9,3 |
| 12 | 7,6 | 7,6 | 8,2 |
| 13 | 7,7 | 7,7 | 9,6 |
| 14 | 6,6 | 6,6 | 6,7 |
| 15 | 8,3 | 6,9 | 8,8 |
| 16 | 8,7 | 7,3 | 9,0 |
| 17 | 7,3 | 7,3 | 8,6 |
| 18 | 8,2 | 6,9 | 7,8 |
| 19 | 7,5 | 7,5 | 8,0 |
| 20 | 7,0 | 5,2 | 8,7 |
| 21 | < 5 | < 5 | 8,3 |

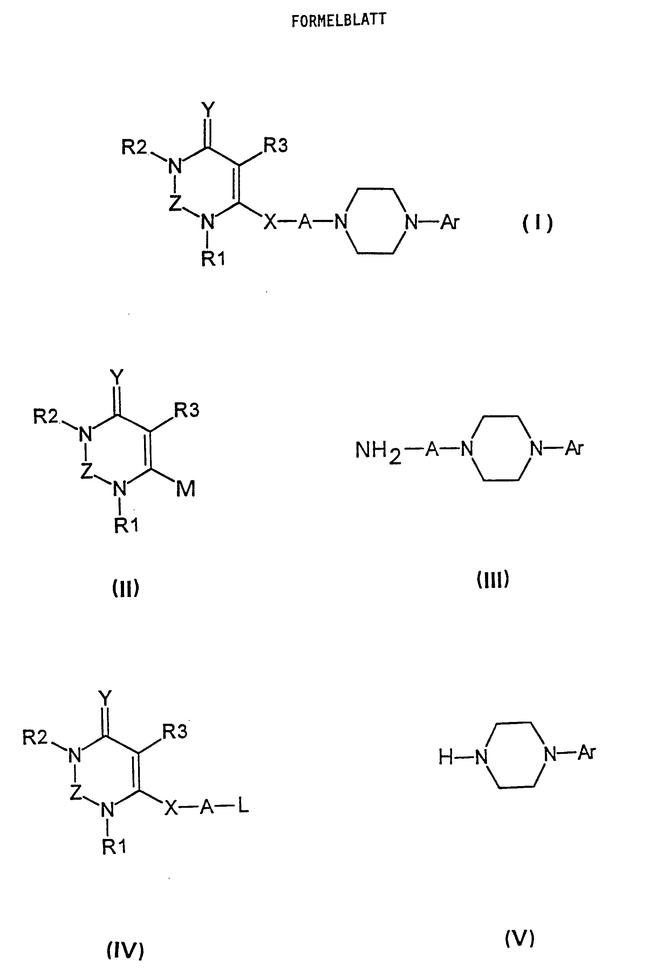

## Patentansprüche

1. Verbindungen der Formel I, 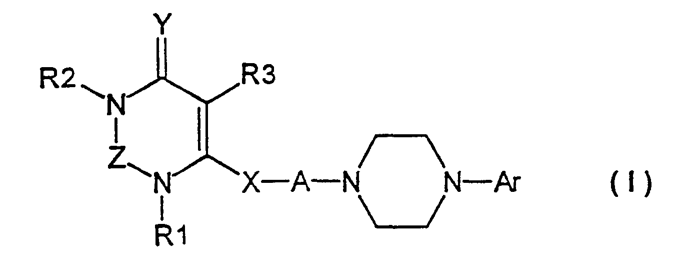 worin
R1 1-4C-Alkyl bedeutet,
R2 1-4C-Alkyl bedeutet,
R3 Wasserstoff (H), 1-7C-Alkyl, 3-7C-Alkenyl, 1-4C-Alkoxy, Halogen, Halo-1-4C-alkyl, Cyan-1-4C-alkyl, 1-4C-Alkoxycarbonyl, Nitro, Hydroxyiminomethyl, Methoxyiminomethyl oder eine Gruppe -CH₂-RI bedeutet, worin RI den in der Formel I an den Substituenten R3 gebundenen Rest bedeutet,
A einen geradkettigen oder verzweigten 1-5C-Alkylenrest bedeutet,
Ar einen durch R4, R5 und R6 substituierten Phenylrest bedeutet, worin
R4 Wasserstoff, Halogen, Nitro, Trifluormethyl, Hydroxy, 1-4C-Alkoxy, ganz oder teilweise durch Fluor substituiertes 1-4C-Alkoxy, Amino oder 1-4C-Alkylcarbonylamino bedeutet,
R5 Wasserstoff, Halogen oder 1-4C-Alkoxy bedeutet und
R6 Wasserstoff oder 1-4C-Alkoxy bedeutet, oder worin
R4 und R5 zueinander orthoständig sind und gemeinsam einen 1-Hydroxymethyl-ethylendioxyrest [-O-CH(CH₂OH)-CH₂-O-] bedeuten und
R6 Wasserstoff bedeutet,
X die Gruppierung NH oder CO-NH bedeutet,
Y Sauerstoff (0) oder Schwefel (S) bedeutet und
Z CO oder SO₂ bedeutet,
und ihre Salze,
wobei
R3 nicht Wasserstoff (H), 1-7C-Alkyl, Halogen oder Nitro bedeutet, wenn
R4 Wasserstoff, Halogen, 1-4C-Alkoxy oder Trifluormethyl,
R5 Wasserstoff, Halogen oder 1-4C-Alkoxy,
R6 Wasserstoff oder 1-4C-Alkoxy,
X die Gruppierung NH,
Y Sauerstoff (0) und
Z CO bedeutet.

2. Verbindungen der Formel I nach Anspruch 1, worin
R1 1-4C-Alkyl bedeutet,
R2 1-4C-Alkyl bedeutet,
R3 Wasserstoff (H), 1-4C-Alkyl, 3-4C-Alkenyl, 1-4C-Alkoxy, Cyan-1-4C-alkyl, 1-4C-Alkoxycarbonyl, Hydroxyiminomethyl, Methoxyiminomethyl oder eine Gruppe -CH₂-RI bedeutet, worin RI den in der Formel I an den Substituenten R3 gebundenen Rest bedeutet,
A einen geradkettigen 2-4C-Alkylenrest bedeutet,
Ar einen durch R4, R5 und R6 substituierten Phenylrest bedeutet, worin
R4 Wasserstoff, Halogen, Nitro, Hydroxy, 1-4C-Alkoxy, ganz oder teilweise durch Fluor substituiertes 1-4C-Alkoxy, Amino oder 1-4C-Alkylcarbonylamino bedeutet,
R5 Wasserstoff, Halogen oder 1-4C-Alkoxy bedeutet und
R6 Wasserstoff oder 1-4C-Alkoxy bedeutet, oder worin
R4 und R5 zueinander orthoständig sind und gemeinsam einen 1-Hydroxymethyl-ethylendioxyrest [-O-CH(CH₂OH)-CH₂-O-1 bedeuten und
R6 Wasserstoff bedeutet,
X die Gruppierung NH oder CO-NH bedeutet,
Y Sauerstoff (0) oder Schwefel (S) bedeutet und
Z CO oder SO₂ bedeutet,
und ihre Salze,
wobei
R3 nicht Wasserstoff (H) oder 1-4C-Alkyl bedeutet, wenn
R4 Wasserstoff, Halogen oder 1-4C-Alkoxy,
R5 Wasserstoff, Halogen oder 1-4C-Alkoxy,
R6 Wasserstoff oder 1-4C-Alkoxy,
X die Gruppierung NH,
Y Sauerstoff (O) und
Z CO bedeutet.

3. Verbindungen der Formel I nach Anspruch 1, worin
R1 1-4C-Alkyl bedeutet,
R2 1-4C-Alkyl bedeutet,
R3 Wasserstoff (H), 1-4C-Alkyl, 3-4C-Alkenyl, 1-4C-Alkoxy, Cyan-1-4C-alkyl, 1-4C-Alkoxycarbonyl, Hydroxyiminomethyl, Methoxyiminomethyl oder eine Gruppe -CH₂-RI bedeutet, worin RI den in der Formel I an den Substituenten R3 gebundenen Rest bedeutet,
A einen Trimethylenrest bedeutet,
Ar einen durch R4, R5 und R6 substituierten Phenylrest bedeutet, worin
R4 Wasserstoff, Halogen, Nitro, Hydroxy, 1-4C-Alkoxy oder ganz oder teilweise durch Fluor substituiertes 1-4C-Alkoxy bedeutet,
R5 Wasserstoff, Halogen oder 1-4C-Alkoxy bedeutet und
R6 Wasserstoff oder 1-4C-Alkoxy bedeutet, oder worin
R4 und R5 zueinander orthoständig sind und gemeinsam einen 1-Hydroxymethyl-ethylendioxyrest [-O-CH(CH₂OH)-CH₂-O-] bedeuten und
R6 Wasserstoff bedeutet.
X die Gruppierung NH oder CO-NH bedeutet,
Y Sauerstoff (O) bedeutet und
Z CO bedeutet,
und ihre Salze,
wobei
R3 nicht Wasserstoff (H) oder 1-4C-Alkyl bedeutet, wenn
R4 Wasserstoff, Halogen oder 1-4C-Alkoxy,
R5 Wasserstoff, Halogen oder 1-4C-Alkoxy,
R6 Wasserstoff oder 1-4C-Alkoxy und
X die Gruppierung NH bedeutet.

4. 1,3,5-Trimethyl-6-[[3-[4-((2,3-dihydro-2-hydroxymethyl)-1,4-benzodioxin-5-yl)-1 -piperazinyl]propyl]amino]-2,4(1H,3H)-pyrimidindion und seine Salze.

5. Arzneimittel enthaltend eine oder mehrere Verbindungen der Formel I nach Anspruch 1 und/oder ihre pharmakologisch verträglichen Salze.

6. Verbindungen der Formel I nach Anspruch 1 und/oder ihre pharmakologisch verträglichen Salze zur Anwendung bei der Behandlung von Krankheiten, die durch 5-HT_{1A}-Agonisten bzw. α₁-Adrenozeptorenblocker therapierbar sind.

7. Verwendung von Verbindungen der Formel I nach Anspruch 1 und ihren pharmakologisch verträglichen Salzen zur Herstellung von Arzneimitteln für die Behandlung von Krankheiten, die durch 5-HT_{1A}-Agonisten bzw. α₁-Adrenozeptorenblocker therapierbar sind.

8. Verbindungen der Formel I nach Anspruch 1 und/oder ihre pharmakologisch verträglichen Salze zur Anwendung bei der Behandlung und/oder Prophylaxe von Krankheiten, die auf zentralnervösen Störungen beruhen.

9. Verbindungen der Formel I nach Anspruch 1 und/oder ihre pharmakologisch verträglichen Salze zur Anwendung bei der Behandlung und/oder Prophylaxe von Krankheiten, die im Zusammenhang mit einer erhöhten Muskelkontraktion im Urogenitaltrakt (neurogene Blase, Prostatahypertrophie etc.) stehen.

## Claims

1. A compound of the formula I 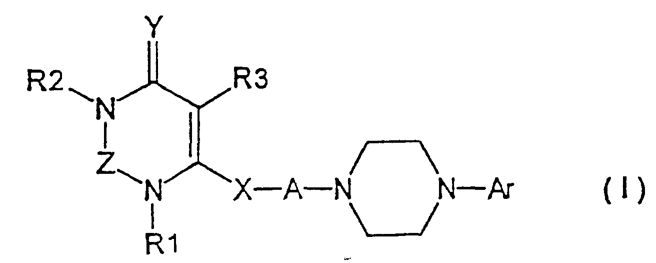 in which
R1 is 1-4C-alkyl,
R2 is 1-4C-alkyl,
R3 is hydrogen (H), 1-7C-alkyl, 3-7C-alkenyl, 1-4C-alkoxy, halogen, halo-1-4C-alkyl, cyano-1-4C-alkyl, 1-4C-alkoxycarbonyl, nitro, hydroxyiminomethyl, methoxyiminomethyl or a group -CH₂-RI, in which RI is the radical bonded to the substituent R3 in the formula I,
A is a straight-chain or branched 1-5C-alkylene radical,
Ar is a phenyl radical substituted by R4, R5 and R6, in which
R4 is hydrogen, halogen, nitro, trifluoromethyl, hydroxyl, 1-4C-alkoxy, completely or partly fluorine-substituted 1-4C-alkoxy, amino or 1-4C-alkylcarbonylamino,
R5 is hydrogen, halogen or 1-4C-alkoxy and
R6 is hydrogen or 1-4C-alkoxy, or in which
R4 and R5 are ortho to one another and together are a 1-hydroxymethylethylenedioxy radical [-O-CH(CH₂OH)-CH₂-O-] and
R6 is hydrogen,
X is the group NH or CO-NH,
Y is oxygen (O) or sulfur (S) and
Z is CO or SO₂,
and their salts,
where
R3 is not hydrogen (H), 1-7C-alkyl, halogen or nitro if
R4 is hydrogen, halogen, 1-4C-alkoxy or trifluoromethyl,
R5 is hydrogen, halogen or 1-4C-alkoxy,
R6 is hydrogen or 1-4C-alkoxy,
X is the group NH,
Y is oxygen (O) and
Z is CO.

2. A compound of the formula I as claimed in claim 1, in which
R1 is 1-4C-alkyl,
R2 is 1-4C-alkyl,
R3 is hydrogen (H), 1-4C-alkyl, 3-4C-alkenyl, 1-4C-alkoxy, cyano-1-4C-alkyl, 1-4C-alkoxycarbonyl, hydroxyiminomethyl, methoxyiminomethyl or a group -CH₂-RI, in which RI is the radical bonded to the substituent R3 in the formula I,
A is a straight-chain 2-4C-alkylene radical,
Ar is a phenyl radical substituted by R4, R5 and R6, in which
R4 is hydrogen, halogen, nitro, hydroxyl, 1-4C-alkoxy, completely or partly fluorine-substituted 1-4C-alkoxy, amino or 1-4C-alkylcarbonylamino,
R5 is hydrogen, halogen or 1-4C-alkoxy and
R6 is hydrogen or 1-4C-alkoxy, or in which
R4 and R5 are ortho to one another and together are a 1-hydroxymethylethylenedioxy radical [-O-CH(CH₂OH)-CH₂-O-] and
R6 is hydrogen,
X is the group NH or CO-NH,
Y is oxygen (O) or sulfur (S) and
Z is CO or SO₂,
and their salts,
where
R3 is not hydrogen (H) or 1-4C-alkyl, if
R4 is hydrogen, halogen or 1-4C-alkoxy,
R5 is hydrogen, halogen or 1-4C-alkoxy,
R6 is hydrogen or 1-4C-alkoxy,
X is the group NH,
Y is oxygen (O) and
Z is CO.

3. A compound of the formula I as claimed in claim 1, in which
R1 is 1-4C-alkyl,
R2 is 1-4C-alkyl,
R3 is hydrogen (H), 1-4C-alkyl, 3-4C-alkenyl, 1-4C-alkoxy, cyano-1-4C-alkyl, 1-4C-alkoxycarbonyl, hydroxyiminomethyl, methoxyiminomethyl or a group -CH₂-RI, in which RI is the radical bonded to the substituent R3 in the formula I,
A is a trimethylene radical,
Ar is a phenyl radical substituted by R4, R5 and R6, in which
R4 is hydrogen, halogen, nitro, hydroxyl, 1-4C-alkoxy, or completely or partly fluorine-substituted 1-4C-alkoxy.
R5 is hydrogen, halogen or 1-4C-alkoxy and
R6 is hydrogen or 1-4C-alkoxy, or in which
R4 and R5 are ortho to one another and together are a 1-hydroxymethylethylenedioxy radical [-O-CH(CH₂OH)-CH₂-O-] and
R6 is hydrogen,
X is the group NH or CO-NH,
Y is oxygen (O) and
Z is CO,
and their salts,
where
R3 is not hydrogen (H) or 1-4C-alkyl, if
R4 is hydrogen, halogen or 1-4C-alkoxy,
R5 is hydrogen, halogen or 1-4C-alkoxy,
R6 is hydrogen or 1-4C-alkoxy, and
X is the group NH.

4. 1,3,5-Trimethyl-6-[[3-[4-((2,3-dihydro-2-hydroxymethyl)-1,4-benzodioxin-5-yl)-1-piperazinyl]amino]-2,4(1H,3H)-pyrimidinedione and its salts.

5. A medicament comprising one or more compounds of the formula I as claimed in claim 1 and/or its pharmacologically tolerable salts.

6. A compound of the formula I as claimed in claim 1 and/or its pharmacologically tolerable salts for use in the treatment of illnesses which can be treated by 5-HT_{1A} agonists or α₁ adrenoceptor blockers.

7. The use of compounds of the formula I as claimed in claim 1 and their pharmacologically tolerable salts for the production of medicaments for the treatment of illnesses which can be treated by 5-HT_{1A} agonists or α₁ adrenoceptor blockers.

8. A compound of the formula I as claimed in claim 1 and/or its pharmacologically tolerable salts for use in the treatment and/or prophylaxis of illnesses which are based on central nervous disorders.

9. A compound of the formula I as claimed in claim 1 and/or its pharmacologically tolerable salts for use in the treatment and/or prophylaxis of illnesses which are connected with increased muscle contraction in the urogenital tract (neurogenic bladder, prostate hypertrophy, etc.)

## Revendications

1. Composés de formule (I) 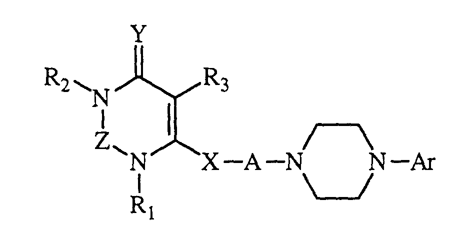 dans laquelle
R₁ représente un groupe alkyle en C₁₋₄,
R₂ représente un groupe alkyle en C₁₋₄,
R₃ représente un atome d'hydrogène (H), un groupe alkyle en C₁₋₇, alcényle en C₃₋₇, alcoxy en C₁₋₄, halogéno, halogéno-alkyle en C₁₋₄, cyano-(alkyle en C₁₋₄), (alcoxy en C₁₋₄)-carbonyle, nitro, hydroxyiminométhyle, méthoxyiminométhyle ou un groupe -CH₂-RI où RI représente le résidu lié au substituant R₃ dans la formule (I),
A représente un résidu alkylène en C₁₋₅, linéaire ou ramifié,
Ar représente un résidu phényle portant les substituants R₄, R₅ et R₆, où
R₄ représente un atome d'hydrogène ou d'halogène, un groupe nitro, trifluorométhyle, hydroxy, alcoxy en C₁₋₄, alcoxy en C₁₋₄ totalement ou partiellement fluoré, amino ou (alkyle en C₁₋₄)-carbonylamino,
R₅ représente un atome d'hydrogène ou d'halogène ou un groupe alcoxy en C₁₋₄, et
R₆ représente un atome d'hydrogène ou un groupe alcoxy en C₁₋₄, ou
R₄ et R₅, en position ortho l'un par rapport à l'autre, représentent conjointement un résidu 1-hydroxyméthyléthylènedioxy [-O-CH(CH₂OH)-CH₂-O-], et
R₆ représente un atome d'hydrogène,
X représente un groupe NH ou CO-NH,
Y représente un atome d'oxygène (O) ou de soufre (S) et
Z représente un groupe CO ou SO₂,
et les sels de ces composés,
avec la réserve que
R₃ ne représente pas un atome d'hydrogène (H), un groupe (alkyle en C₁₋₇), un atome d'halogène ou un groupe nitro, lorsque
R₄ représente un atome d'hydrogène ou d'halogène ou un groupe alcoxy en C₁₋₄ ou trifluorométhyle,
R₅ représente un atome d'hydrogène ou d'halogène ou un groupe alcoxy en C₁₋₄,
R₆ représente un atome d'hydrogène ou un groupe alcoxy en C₁₋₄,
X représente un groupe NH,
Y un atome d'oxygène (O) et
Z un groupe CO.

2. Composés de formule (I) selon la revendication 1 dans lesquels
R₁ représente un groupe alkyle en C₁₋₄,
R₂ représente un groupe alkyle en C₁₋₄,
R₃ représente un atome d'hydrogène (H), un groupe alkyle en C₁₋₇, alcényle en C₃₋₄, alcoxy en C₁₋₄, cyano-(alkyle en C₁₋₄), (alcoxy en C₁₋₄)-carbonyle, hydroxyiminométhyle, méthoxyiminométhyle ou un groupe de formule -CH₂-RI où RI représente le résidu lié au substituant R₃ dans la formule (I),
A représente un résidu alkylène en C₂₋₅ linéaire,
Ar représente un résidu phényle portant les substituants R₄, R₅ et R₆, où
R₄ représente un atome d'hydrogène ou d'halogène, un groupe nitro, hydroxy, alcoxy en C₁₋₄, alcoxy en C₁₋₄ totalement ou partiellement fluoré, amino ou (alkyle en C₁₋₄)-carbonylamino,
R₅ représente un atome d'hydrogène ou d'halogène ou un groupe alcoxy en C₁₋₄, et
R₆ représente un atome d'hydrogène ou un groupe alcoxy en C₁₋₄, ou
R₄ et R₅, en position ortho l'un par rapport à l'autre, représentent conjointement un résidu 1-hydroxyméthyléthylènedioxy [-O-CH(CH₂OH)-CH₂-O-], et
R₆ représente un atome d'hydrogène,
X représente un groupe NH ou CO-NH,
Y représente un atome d'oxygène (O) ou de soufre (S) et
Z représente un groupe CO ou SO₂,
et les sels de ces composés,
avec la réserve que
R₃ ne représente pas un atome d'hydrogène (H) ou un groupe (alkyle en C₁₋₄), lorsque
R₄ représente un atome d'hydrogène ou d'halogène ou un groupe alcoxy en C₁₋₄,
R₅ représente un atome d'hydrogène ou d'halogène ou un groupe alcoxy en C₁₋₄,
R₆ représente un atome d'hydrogène ou un groupe alcoxy en C₁₋₄,
X représente un groupe NH,
Y un atome d'oxygène (O) et
Z un groupe CO.

3. Composés de formule (I) selon la revendication 1 dans lesquels
R₁ représente un groupe alkyle en C₁₋₄,
R₂ représente un groupe alkyle en C₁₋₄,
R₃ représente un atome d'hydrogène (H), un groupe alkyle en C₁₋₇, alcényle en C₃₋₄, alcoxy en C₁₋₄, cyano-(alkyle en C₁₋₄), (alcoxy en C₁₋₄)-carbonyle, hydroxyiminométhyle, méthoxyiminométhyle ou un groupe de formule -CH₂-RI où RI représente le résidu lié au substituant R₃ dans la formule (I),
A représente un résidu triméthylène,
Ar représente un résidu phényle portant des substituants R₄, R₅ et R₆, où
R₄ représente un atome d'hydrogène ou d'halogène, un groupe nitro, hydroxy, alcoxy en C₁₋₄ ou alcoxy en C₁₋₄ totalement ou partiellement fluoré,
R₅ représente un atome d'hydrogène ou d'halogène ou un groupe alcoxy en C₁₋₄, et
R₆ représente un atome d'hydrogène ou un groupe alcoxy en C₁₋₄, ou
R₄ et R₅, en position ortho l'un par rapport à l'autre, représentent conjointement un résidu 1-hydroxyméthyléthylènedioxy [-O-CH(CH₂OH)-CH₂-O-], et
R₆ représente un atome d'hydrogène,
X représente un groupe NH ou CO-NH,
Y représente un atome d'oxygène (O) et
Z représente un groupe CO,
et les sels de ces composés,
avec la réserve que
R₃ ne représente pas un atome d'hydrogène (H), un groupe alkyle en C₁₋₄, lorsque
R₄ est un atome d'hydrogène ou d'halogène ou un groupe alcoxy en C₁₋₄,
R₅ représente un atome d'hydrogène ou d'halogène ou un groupe alcoxy en C₁₋₄,
R₆ représente un atome d'hydrogène ou un groupe alcoxy en C₁₋₄,
X représente un groupe NH.

4. 1,3,5-triméthyl-6-[[3-[-((2,3-dihydrogéno-2-hydroxyméthyl)-1,4-benzodioxin-5-yl)-1-pipérazinyl]propyl] amino] -2,4-(1H,3H)-pyrimidine-dione et les sels correspondants.

5. Médicament contenant un ou plusieurs composés de formule (I) selon la revendication 1 et/ou leurs sels pharmacologiquement acceptables.

6. Composés de formule (I) selon la revendication 1 et/ou leurs sels pharmacologiquement acceptables pour le traitement de maladies susceptibles d'être traitées par des agonistes 5-HT_{1A} ou des agents bloquant les récepteurs adrénergiques α₁.

7. Utilisation de composés de formule (I) selon la revendication 1 et de leurs sels pharmacologiquement acceptables pour la fabrication de médicaments destinés au traitement de maladies susceptibles d'être traitées par des agonistes 5-HT_{1A} ou des agents bloquant les récepteurs adrénergiques α₁.

8. Composés de formule ()I selon la revendication 1 et/ou leurs sels pharmacologiquement acceptables pour le traitement et/ou la prévention de maladies dues à des dysfonctionnements du système nerveux central.

9. Composés de formule (I) selon la revendication 1 et/ou leur sels pharmacologiquement acceptables pour le traitement et/ou la prévention de maladies liées à une contraction musculaire anormalement élevée dans le tractus urogénital (vessie neurogène, hypertrophie de la prostate etc.).
